Europäisches Patentamt

European Patent Office

Office européen des brevets

(19)

(11) Veröffentlichungsnummer: **0 293 659**
**A2**

(12) **EUROPÄISCHE PATENTANMELDUNG**

(21) Anmeldenummer: **88107780.4**

(22) Anmeldetag: **14.05.88**

(51) Int. Cl.4: **C07D 307/32**

(30) Priorität: **04.06.87 DE 3718687**

(43) Veröffentlichungstag der Anmeldung:
**07.12.88 Patentblatt 88/49**

(84) Benannte Vertragsstaaten:
**BE CH DE ES FR GB IT LI NL**

(71) Anmelder: **HÜLS AKTIENGESELLSCHAFT**
**- RSP Patente / PB 15 - Postfach 13 20**
**D-4370 Marl 1(DE)**

(72) Erfinder: **Gude, Fritz, Dr.**
**Zur Emschermulde 24**
**D-4690 Herne 2(DE)**
Erfinder: **Winter, Rolf**
**Auf der Bovenhorst 10**
**D-4270 Dorsten 1(DE)**

(54) **Verfahren zur Herstellung von Gamma-Undecalacton aus Omega-Undecylensäure.**

(57) Bekannte Verfahren arbeiten mit großen Mengen an konzentrierter Schwefelsäure als Katalysator, wobei sich unter Dunkelfärbung des Produkts verschiedene unerwünschte Nebenreaktionen abspielen und mehrere Wäschen erfolgen müssen. Das neue Verfahren soll zu einem sauberen Produkt unter Reduzierung der Wäschen führen.

Im vorliegenden Verfahren werden stark saure, bei Reaktionsbedingungen stabile Ionenaustauscher eingesetzt. Bei guter Ausbeute ist das Verfahren weniger aufwendig.

Herstellung von Gamma-Undecalacton für die Riechstoffindustrie.

EP 0 293 659 A2

## Verfahren zur Herstellung von Gamma-Undecalacton aus Omega-Undecylensäure

Die durch Spaltung von Rizinusöl zugängliche Omega-Undecylensäure reagiert durch Isomerisierung der Doppelbindung mit konzentrierter Schwefelsäure und Ringschluß zum Gamma-Undecalacton, das als pfirsichartig riechender Duftstoff in Parfümierungen von Cremes und Lippenstiften Eingang gefunden hat.

Die Methode der Doppelbindungsisomerisierung von Omega-Undecylensäure und der darauf folgende Ringschluß zu Gamma-Undecalacton mit Schwefelsäure samt der Aufarbeitung des Duftstoffes stammt von E. Blaise und L. Huillon (Cosmetics, Flagrances und Flowers, Novox Inc., Publisher, Whiting, New Jersey, 1982, 1. Auflage, Seite 422). Abgesehen von der notwendig großen Menge konzentrierter Schwefelsäure - die Hälfte des Gewichtes der eingesetzten Undecylensäure - besitzt die Methode eine Reihe gravierender Nachteile, wie sich bei der Nacharbeit herausstellte.

Während der 20stündigen Reaktion zur Isomerisierung der Doppelbindung und Lactonisierung bei einer Temperatur von 100 °C, spaltet sich, deutlich wahrnehmbar, Schwefeldioxid unter Verfärbung der Substanz nach tiefschwarz ab. Beides deutet auf unerwünschte Nebenreaktionen hin, die die Ursache für Komplikationen während der Aufarbeitung sind. Anschließend ist zur Entfernung der Hauptmenge an Schwefelsäure eine zweimalige intensive Wasserwäsche notwendig. Dabei fällt auf, daß sich auch die wäßrige Schicht tiefschwarz färbt, so daß eine Trennung der Schichten sehr erschwert wird. Die Bildung einer kräftigen Emulsion verzögert darüber hinaus diesen Waschvorgang erheblich. Nach Verdünnung mit Toluol erfolgt eine weitere Wäsche mit überschüssiger 10 %iger Sodalösung, wobei wiederum stundenlang beständige, lästige Emulsionen auftreten. Da eine saubere Trennung der Schichten nicht möglich ist, muß anschließend noch die Toluollösung mit Wasser und die wäßrige Schicht mit Toluol gewaschen werden. Anschließend vereinigt man die Toluolschichten, dampft das Lösungsmittel ab und destilliert den Rückstand im Vakuum. Auf diese Weise erhält man ein übelriechendes Destillat, das wiederum mit Sodalösung, dann mit Natronlauge, gewaschen wird. Nach Trocknen über Natriumsulfat erfolgt eine Fraktionierung über Ätznatronplätzchen. Danach vereinigt man Vorlauf und Nachlauf und gewinnt durch Destillation eine weitere Menge Undecalacton.

Die Autoren bemerken, daß sie auf diese Weise eine Ausbeute von etwa 40 % der Theorie (hochgerechnet auf 100 %ige Undecylensäure) an reinem Undecalacton erhalten hätten. Bei der Nacharbeit wurden bei mehreren Ansätzen allenfalls 20 bis 25 % der Theorie erreicht.

Um dieses umständliche Verfahren zu vereinfachen, Chemikalien einzusparen, die Grenzfläche bei den Wäschen deutlich sichtbar zu machen, die Emulsionsbildung zu vermeiden und den Trennvorgang erheblich abzukürzen, wurde versucht, einen anderen Katalysator zu finden, der anstelle von konzentrierter Schwefelsäure die Isomerisation der Undecylensäure unter Bildung von Undecalacton bewirkt.

Gegenstand der Erfindung ist ein Verfahren zur Herstellung von Gamma-Undecalacton aus Omega-Undecylensäure in Gegenwart saurer Katalysatoren, dadurch gekennzeichnet, daß als Katalysator stark saure, im Reaktionsmedium bei einer Reaktionstemperatur von 100 bis 130 °C beständige Ionentauscher in Mengen von 10 bis 20 Gew.-%, bezogen auf eingesetzte Omega-Undecylensäure, verwendet werden.

Alle angestrebten Verbesserungen des Verfahrens können überraschend durch den erfindungsgemäßen Einsatz des Katalysators erreicht werden, wobei man die Reaktionszeit von 20 Stunden bis auf die Hälfte, ohne wesentliche Verminderung der Ausbeute, reduzieren kann. Nach Abfiltrieren des Austauschers stellt man durch Gaschromatographie überraschend einen Umsatz von 70 bis 80 % der Theorie fest. Bei der anschließenden Aufarbeitung entfällt die Wasserwäsche, die nach dem Bezugsverfahren zur Entfernung der Schwefelsäure notwendig ist. Auf einige Verfahrensschritte kann verzichtet werden. Die Wäschen mit wäßrigen Lösungen können wegen der einwandfreien Trennung der Schichten ohne jede Emulsionsbildung und praktisch ohne Verfärbung in erheblich kürzerer Zeit durchgeführt werden. Die Ausbeute an Undecalacton läßt sich auf diese Weise sogar auf etwa 44 % der Theorie steigern.

Der Katalysator kann ohne wesentliche Ausbeuteeinschränkungen noch einige Male wiederverwendet werden, evtl. unter Zusatz von 10 bis 20 % frischem Material.

Als Katalysator für die Reaktion gemäß dieser Erfindung kommen alle stark sauren Ionenaustauscher in Betracht, die in organischen Lösungsmitteln bei etwa 100 bis 130 °C beständig und unlöslich sind. Als wirksame Gruppierung dient im allgemeinen ein Sulfosäurerest.

Geeignete, handelsübliche Ionenaustauschertypen sind z. B. LEWATIT SPC 118[R] und LEWATIT SPC 108[R] der Bayer AG, Leverkusen, auf Basis Styrol-Divinylbenzol sowie AMBERLIST 15[R] der Firma Rohm & Haas, Philadelphia, auf Kunstharzbasis. Dies soll aber keine Beschränkung der brauchbaren Katalysatortypen gemäß dieser Erfindung darstellen.

Beispiele

1. (Vergleichsversuch nach Blaise und Huillon) Nacharbeit

In 600 g Undecylensäure (Reinheit 99,2 %) tropft man langsam unter Kühlung des Reaktionsgefäßes und Rührung 300 g 92 %ige Schwefelsäure in der Weise zu, daß sich die Temperatur unter 50 °C hält. Anschließend wird langsam auf 95 bis 100 °C erhitzt und die Temperatur unter weiterem Rühren 20 Stunden gehalten. Während dieser Operation verfärbt sich das Reaktionsgemisch unter Abspalten von Schwefeldioxid nach tiefschwarz. Schließlich wird auf 50 °C abgekühlt.

Nun fügt man 600 g kaltes Wasser hinzu und rührt eine halbe Stunde mit geringer Drehzahl. Anschließend läßt man Absitzen, trennt die wäßrige Phase ab und wiederholt den Waschvorgang noch einmal. Beim Abtrennen ist die Phasengrenze - schlecht zu erkennen, weil beide Schichten dunkel gefärbt sind. Das Waschwasser wird verworfen. Jetzt versetzt man das Reaktionsgemisch mit 500 g Toluol und einer 10 %igen wäßrigen Sodalösung bis zur alkalischen Reaktion, wobei starke Kohlendioxidbildung auftritt. Man rührt langsam und heizt bis auf 80 °C, um die Trennung der beiden Schichten zu erleichtern und läßt die Carbonatlösung ab. Nun wird die Toluollösung mit Wasser gewaschen, wobei die Reaktion alkalisch bleiben muß, erwärmt wiederum auf etwa 80 °C und läßt nach Trennung der Phase das Wasser ab. Man vereinigt die alkalischen Wässer und extrahiert sie bei 80 °C mit 200 g Toluol, um noch etwas Undecalacton zu gewinnen.

Nach Vereinigung der Toluollösung destilliert man das Lösungsmittel mit einem 1 000 cm³ fassenden Kolben bei Normaldruck bis 130 °C ab. Dann wird der Rückstand schnell unter gutem Vakuum (etwa 0,1 mbar) destilliert, bis die Temperatur im Sumpf 200 °C erreicht. Das Destillat hat eine gelbe Farbe und besitzt einen stechenden Geruch.

Zur weiteren Reinigung wäscht man nochmal mit 5 %iger Sodalösung bei 80 °C, trennt sie ab und schüttelt in der Kälte mit 1 %iger Natronlauge. Anschließend wird über wasserfreiem Natriumsulfat getrocknet und bei einem Vakuum bei 0,05 bis 0,1 mbar fraktioniert, wobei der Hauptlauf zwischen 100 bis 115 °C übergeht. Vor- und Hauptlauf vereinigt man und destilliert unter gleichen Bedingungen, um möglichst viel Undecalacton zu gewinnen. Die Ausbeute beträgt 20 bis 25 % der Theorie an Undecalacton.

## 2. Beispiele gemäß der Erfindung

a) Undecylensäure (Reinheit 99,2 %) wird zur Herstellung des Undecalactons mit 20 % AMBERLIST 15[R] 20 Stunden unter Stickstoff und Rühren auf 130 °C erhitzt. Anschließend filtriert man den Katalysator ab und wäscht ihn mit Toluol. Dieser Toluolextrakt wird zusammen mit dem Rohlacton aufgearbeitet und evtl. noch Toluol hinzugefügt, so daß eine etwa 50 %ige Lösung entsteht.

In Anlehnung an die Vorschrift von Blaise und Huillon wird anschließend mit überschüssiger 10 %iger Sodalösung bei 80 °C gewaschen.

Der Sodawäsche folgt ein Ausschütteln mit Wasser. Bei allen diesen Waschoperationen tritt praktisch keine Emulsionsbildung auf. Die Lösungen sind fast farblos. Nun destilliert man die organische Substanz rasch bei etwa 1 mbar über. Das Rohdestillat wird mit 5 %iger Sodalösung bei 80 °C und anschließend in der Kälte mit 1 %iger Natronlauge gewaschen. Nach Trocknen des Rohlactons über wasserfreiem Natriumsulfat erfolgt die Vakuumfraktionierung bei < 0,5 mbar über NaOH-Plätzchen. Der Hauptlauf geht bei 100 bis 115 °C über. Vor- und Nachlauf werden vereinigt und nochmal fraktioniert, um bei dem angegebenen Siedepunkt noch eine weitere Menge Undecalacton zu erhalten. Insgesamt gewinnt man etwa 44 % der Theorie an Undecalacton mit Riechstoffqualität.

b) Der aus Versuch 2 zurückgewonnene, mit Toluol extrahierte Katalysator wird bei einem weiteren Versuch nochmal - wie vorher beschrieben - eingesetzt. Die Reaktionszeit beträgt 10 Stunden. Nach der Aufarbeitung erhält man eine Undecalacton-Ausbeute von 40,5 % der Theorie.

c) Bei einem weiteren Versuch mit 10 % LEWATIT SPC 118[R] als Katalysator kann man unter gleichen Bedingungen, wie unter 2) beschrieben, eine Ausbeute von 39 % der Theorie an Undecalacton erreichen.

## Ansprüche

Verfahren zur Herstellung von Gamma-Undecalacton aus Omega-Undecylensäure in Gegenwart saurer Katalysatoren,
dadurch gekennzeichnet,
daß als Katalysator stark saure, im Reaktionsmedium bei einer Reaktionstemperatur von 100 bis 130 °C beständige Ionentauscher in Mengen von 10 bis 20 Gew.-%, bezogen auf eingesetzte Omega-Undecylensäure, verwendet werden.